# EUROPEAN PATENT APPLICATION

(11) **EP 3 925 603 A1**
(43) Date of publication of application: **22.12.2021**
(21) Application number: 20738059.3
(22) Date of filing: 07.01.2020
(51) Int. Cl.: A61K 31/01, A61K 31/11, A61K 9/00, A61P 37/08, A61P 17/00, A61P 17/02, A61K 8/31, A61K 8/33, A61Q 19/00

(54) **COMPOSITION FOR ALLERGY PREVENTION, ATOPIC DERMATITIS ALLEVIATION OR SKIN REGENERATION, CONTAINING, AS ACTIVE INGREDIENT, UNDECANE OR UNDECANAL**

(30) Priority: 10.01.2019 KR 20190003549
(71) Applicant: Botanicsens, Jeollabuk-do 54871 (KR)
(72) Inventor: PARK, Tae Sun, Seoul 03174 (KR)
(74) Representative: V.O.
(86) International application number: PCT/KR2020/000257
(87) International publication number: WO 2020/145619

(57) **Abstract**

The present invention relates to a composition for allergy prevention, atopic dermatitis alleviation or skin regeneration, containing, as an active ingredient, undecane or undecanal and, more specifically, to a composition for preventing or treating allergic disease or atopic dermatitis and a composition for healing skin wound or promoting skin regeneration, both of which contain, as an active ingredient, at least one selected from a group consisting of undecane, undecanal or pharmaceutically acceptable salts thereof.

## Description

### [Technical Field]

The present application claims priority to Korea Patent Application No. 10-2019-0003549 filed on January 10, 2019, which is incorporated as a reference in its entire content herein.

The present disclosure relates to a composition for allergy prevention, atopic dermatitis alleviation or skin regeneration, containing, as an active ingredient, undecane or undecanal and, more specifically, to a composition for preventing or treating allergic disease or atopic dermatitis and a composition for healing skin wound or promoting skin regeneration, both of which contain, as an active ingredient, at least one selected from a group consisting of undecane, undecanal or pharmaceutically acceptable salts thereof.

### [Background Art]

Atopy is derived from the ancient Greek word 'atopia', which means 'strange, abnormal'. The tendency to genetically induce specific immune-antibody reactions to allergens was first called 'atopy' by Cooke and Coca in 1923. Atopy has been steadily increasing worldwide, and the incidence rate is increasing in more industrially developed countries.

A cause of the development of atopy has not yet been known precisely, but is not limited to one factor, such as family history, changes in diet, penetration of allergic antigen, abnormal skin barrier, etc., and is a combination of several factors. Atopy may mainly develop in infancy and childhood and continue or develop in adulthood. Typical symptoms of atopy appear on the hands, scalp, face, neck, elbows, etc., but have differences in the pattern appearing for each period. The symptoms of atopy in infancy are rough and dry skin, and the development of dermatitis on the outside the limbs, and often appear on the cheeks or forehead, and produce a scab or discharge after scratching with the hands. The symptoms of atopy in childhood mainly appear in the folded areas of arms, legs, neck, etc., rather than the face, and the skin becomes dry. The symptoms of atopy in puberty and adulthood are a thickening of the skin on areas such as the face and hands.

Topical steroids, topical immunomodulators, systemic steroids, systemic immunosuppressants, and antihistamines are used as drugs for treating atopy. The topical steroids are used for severe atopic symptoms, and their use is the most basic method to manage bacterial or viral infections. However, the steroids were introduced in 1950 and have been used for several years, but their long-term use is limited due to problems in the safety and tolerance of the skin depending on the number of uses, concentration, and duration. In addition, the steroids may cause potential side effects such as hypothalamic-pituitary-adrenal (HPA) inhibition and Cushing's syndrome, etc., as well as skin side effects such as skin atrophy, telangiectasia, and steroid acne, so sufficient caution is required when using them.

It is known that tacrolimus and pimecrolimus, local immunomodulatory agents, may be used for a long period of time for the purpose of preventing lesion recurrence due to the relatively small possibility of side effects even when used for a long period of time, unlike existing topical steroids, and tacrolimus and pimecrolimus are suitable for use in the treatment and maintenance therapy for mild atopy. However, tacrolimus may cause side effects such as deterioration in renal function, hand tremors, and hair loss, and pimecrolimus may cause serious side effects such as skin cancer and lymphoma as well as acne and burning. Steroids are suitable for use in acute exacerbation, and topical immunomodulators are suitable for use in the treatment and maintenance therapy of mild atopy, but the safety against side effects has not been secured yet, so there is an urgent need for alternative complementary products.

Accordingly, the present applicant made an effort to develop a material that is effective in alleviating the symptoms of atopic dermatitis with few side effects, and as a result, confirmed that undecane and undecanal relieve the symptoms of atopy, alleviate the inflammatory response and have an anti-allergic and wound healing effect, thereby completing the present disclosure.

### [Related Art Document]

(Patent Document 1) Korea patent application publication No. 10-2018-0128602

### [Disclosure]

### [Technical Problem]

An object of the present disclosure is to provide a pharmaceutical composition for preventing or treating allergic diseases or atopic dermatitis, containing, as an active ingredient, one or more selected from the group consisting of undecane, undecanal, and pharmaceutically acceptable salts thereof.

Another object of the present disclosure is to provide a composition for preventing or treating allergic diseases or atopic dermatitis, containing, as an active ingredient, one or more selected from the group consisting of undecane, undecanal, and pharmaceutically acceptable salts thereof.

Another object of the present disclosure is to provide a pharmaceutical composition for curing skin wound or promoting skin regeneration, containing, as an active ingredient, one or more selected from the group consisting of undecane, undecanal, and pharmaceutically acceptable salts thereof.

Another object of the present disclosure is to provide a quasi-drug for curing skin wound or promoting skin regeneration, containing, as an active ingredient, one or more selected from the group consisting of undecane, undecanal, and pharmaceutically acceptable salts thereof.

Another object of the present disclosure is to provide a cosmetic composition for curing skin wound or promoting skin regeneration, containing, as an active ingredient, one or more selected from the group consisting of undecane, undecanal, and pharmaceutically acceptable salts thereof.

### [Technical Solution]

The present disclosure is to provide a pharmaceutical composition for preventing or treating allergic diseases or atopic dermatitis, containing, as an active ingredient, one or more selected from the group consisting of undecane, undecanal, and pharmaceutically acceptable salts thereof to solve technical problem as disclosed above.

According to one aspect of the present disclosure, the allergic disease may be selected from the group consisting of edema, anaphylaxis, allergic rhinitis, asthma, allergic conjunctivitis, allergic conjunctivitis, allergic dermatitis, contact dermatitis, hives, pruritus, insect allergy, food allergy, and drug allergy, but is not limited thereto.

According to one aspect of the present disclosure, the active ingredient may represent anti-allergic effect, or a preventive or therapeutic effect of atopic dermatitis by reducing the expression of IL-4 (Interleukin-4), IL-13, TNF-α (tumor necrosis factor-alpha), IL-1β, IL-6, or IL-8.

Also, the present disclosure provides a composition for preventing or alleviating allergic diseases or atopic dermatitis, containing, as an active ingredient, one or more selected from the group consisting of undecane, undecanal, and pharmaceutically acceptable salts thereof.

According to one embodiment of the present disclosure, the composition may be a health functional food composition, a cosmetic composition, or a fragrance composition.

Also, the present disclosure provides a pharmaceutical composition for curing skin wound or promoting skin regeneration, containing, as an active ingredient, one or more selected from the group consisting of undecane, undecanal, and pharmaceutically acceptable salts thereof.

In addition, the present disclosure provides a quasi-drug for curing skin wound or promoting skin regeneration, containing, as an active ingredient, one or more selected from the group consisting of undecane, undecanal, and pharmaceutically acceptable salts thereof.

Further, the present disclosure provides a cosmetic composition for curing skin wound or promoting skin regeneration, containing, as an active ingredient, one or more selected from the group consisting of undecane, undecanal, and pharmaceutically acceptable salts thereof.

Furthermore, the present disclosure provides a method for preventing or treating allergic diseases or atopic dermatitis comprising administering or taking a composition containing, as an active ingredient, one or more selected from the group consisting of undecane, undecanal, and pharmaceutically acceptable salts thereof to a subject.

Additionally, the present disclosure provides a use for preventing or alleviating allergic diseases or atopic dermatitis of a composition containing, as an active ingredient, one or more selected from the group consisting of undecane, undecanal, and pharmaceutically acceptable salts thereof.

### [Advantageous Effects]

The composition containing, as an active ingredient, undecane, undecanal, or salts thereof according to the present disclosure, has an effect of alleviating atopic dermatitis caused by allergic reactions, and may be used as a composition for preventing or treating various allergic diseases by reducing the inflammatory response. In addition, the composition according to the present disclosure may have a skin regeneration effect, and thus be usefully used as a wound healing drug or a functional cosmetic for skin regeneration.

### [Description of Drawings]

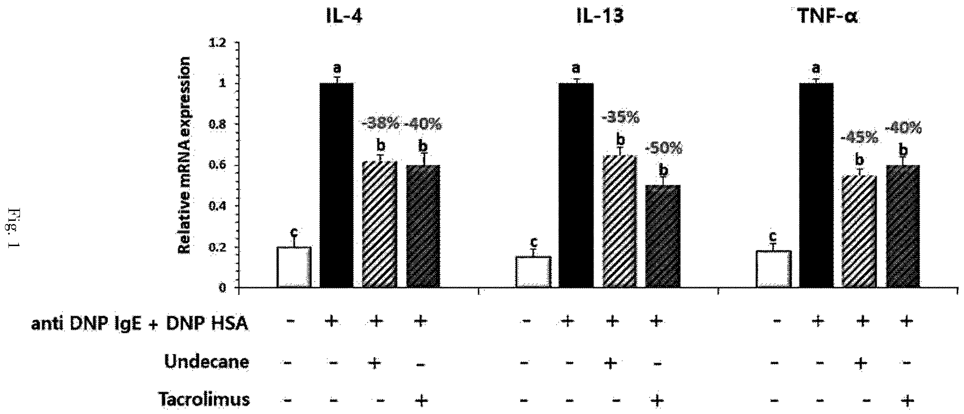

FIG. 1 is a graph showing the expression change of inflammatory cytokine-related molecules (IL-4, IL-13, and TNF-α) in mast cells treated with undecane (each value is mean ± SEM of 3 times obtained from 3 independent wells; letters above bars indicate statistically significant differences at P<0.05).

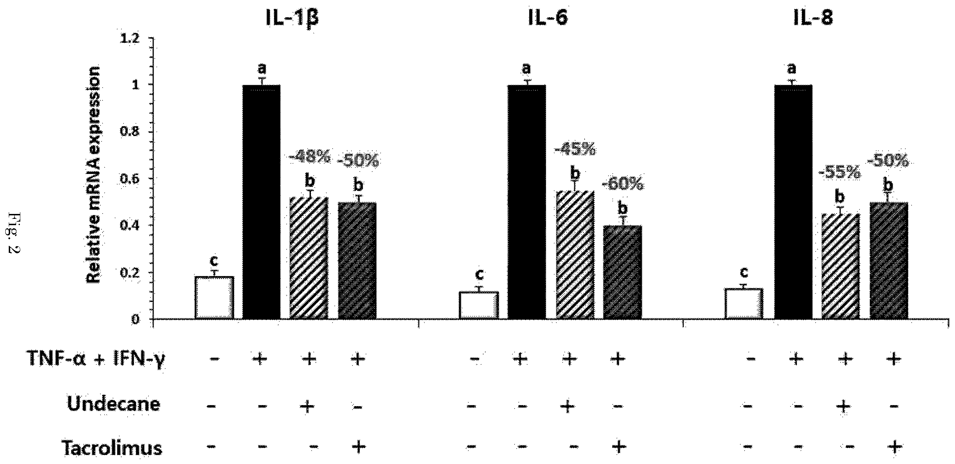

FIG. 2 is a graph showing the expression change of inflammatory cytokine-related molecules (IL-1β, IL-6, and IL-8) in keratinocytes treated with undecane (each value is mean ± SEM of 3 times obtained from 3 independent wells; letters above bars indicate statistically significant differences at P<0.05).

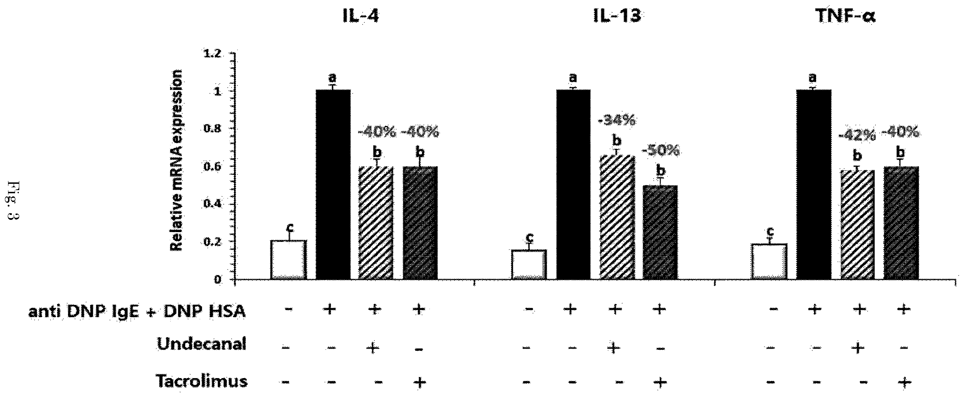

FIG. 3 is a graph showing the expression change of inflammatory cytokine-related molecules (IL-4, IL-13, and TNF-α) in mast cells treated with undecanal (each value is mean ± SEM of 3 times obtained from 3 independent wells; letters above bars indicate statistically significant differences at P<0.05).

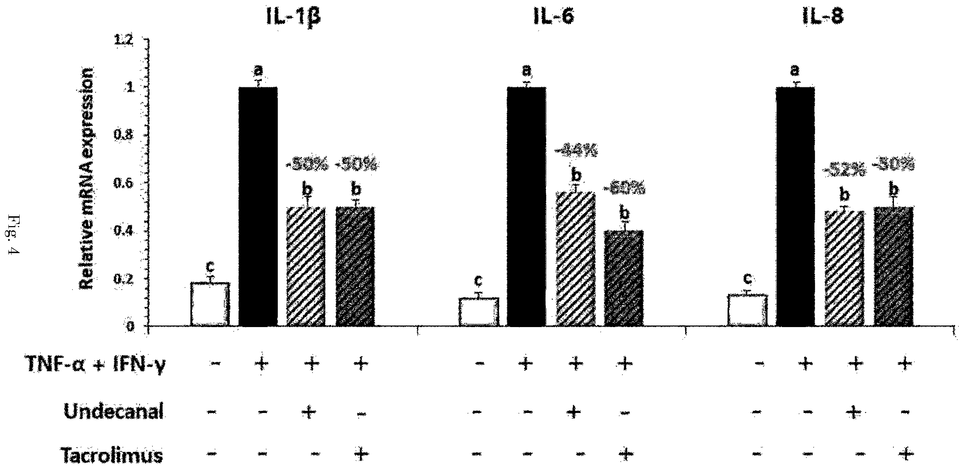

FIG. 4 is a graph showing the expression change of inflammatory cytokine-related molecules (IL-1β, IL-6, and IL-8) in keratinocytes treated with undecanal (each value is mean ± SEM of 3 times obtained from 3 independent wells; letters above bars indicate statistically significant differences at P<0.05).

### [Best Mode]

The present inventors have completed the present disclosure by confirming that undecane and undecanal relieve atopic symptoms, and significantly reduce the expression of IL-4, IL-13, TNF-α, IL-1β, IL-6, or IL-8, which is inflammatory cytokine secreted by the immune response of cells.

Hereinafter, the present disclosure will be described in detail.

The present disclosure provides a composition for preventing or alleviating allergic diseases or atopic dermatitis, containing, as an active ingredient, one or more selected from the group consisting of undecane, undecanal, and pharmaceutically acceptable salts thereof.

Specifically, the undecane is an alkane-based compound, and has a structural formula of C₁₁H₂₄ as represented by the following Formula 1 and a molecular weight of 156.31 g/mol. Undecane is also called by other names such as N-hendecane, hendecane, N-undecane, etc. Undecane is a colorless and transparent liquid component, with a melting point of -26°C and a boiling point of 195-198°C.

Undecane is mainly contained in Dialium guineense (velvet tamarind), Citrus unshiu (tangerine), and Ulva rigida C. Agardh (ulva). Undecane is mainly used as an emollient and is listed as a cosmetic ingredient in the Korean Cosmetics Association.

Undecane is known to be relatively safe in rats, with an LD₅₀ (lethal dose, 50%) value of about 442 ppm/8H for inhalation toxicity.

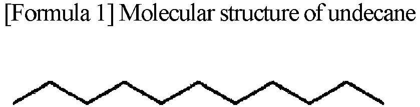

Undecanal is an aldehyde-based compound, and has a structural formula of C₁₁H₂₂O as represented by the following Formula 2 and a molecular weight of 170.3 g/mol. Undecanal is also called by other names such as undecanaldehyde, N-undecanal, undecyl aldehyde, undecylic aldehyde, etc. Undecanal is a pale yellow, transparent liquid component with a melting point of - 3°C and a boiling point of223°C.

Undecanal is a fragrance component mainly contained in Coriander leaf, cerastium candidissimum, herniaria incana, etc., and has a soapy, waxy, and aldehydic-like scent. Undecanal is mainly used as a fragrance component in a flavoring agent, fragrances, cleaning agent, detergent, etc., and has been approved by Flavor and Extract Manufacturers Association (FEMA) and Joint FAO/WHO Expert Committe on Food Additives (JECFA) as safe as flavoring agents and food additives, respectively.

It is known that during acute oral administration of undecanal to rats, an LD₅₀ (lethal dose, 50%) value is > 5,000 mg/kg bw/day, and during chronic oral administration of undecanal to rats, the no-observed-adverse-effect level (NOAEL) is about 1000 mg/kg bw, which is relatively safe.

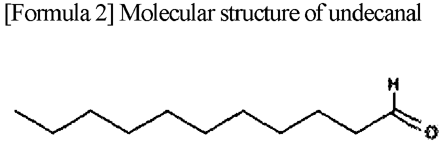

Undecane or undecanal according to the present disclosure may include undecane or undecanal hydrate, undecane or undecanal derivative, etc., within the range having the same efficacy as the undecane or undecanal, and also may include solvates or stereoisomers.

The undecane or undecanal is not particularly limited in the obtaining method, and may use those which are isolated from a plant containing the undecane or undecanal, chemically synthesized by using a known method, or commercially available.

As used herein, the term "cosmetically acceptable salts", "sitologically acceptable salts", "pharmaceutically acceptable salts" or "salts thereof' may be acid addition salts formed by a free acid. Acid addition salts may be prepared by conventional methods, for example by dissolving the compound in an excess of an aqueous acid solution and precipitating the salts with a water-miscible organic solvent such as methanol, ethanol, acetone or acetonitrile. In addition, an equimolar amount of the compound and an acid or alcohol (e.g., glycol monomethyl ether) in water may be heated and then the mixture may be evaporated to dryness, or the precipitated salts may be filtered off with suction.

Example of the free acid may include an inorganic acid or an organic acid. Non-limiting examples of the inorganic acid include hydrochloric acid, phosphoric acid, sulfuric acid, nitric acid, tartaric acid, etc., and these may be used in alone or in combination of two or more as described above. Non-limiting examples of the organic acid include methanesulfonic acid, p-toluenesulfonic acid, acetic acid, trifluoroacetic acid, maleic acid, succinic acid, oxalic acid, benzoic acid, tartaric acid, fumaric acid, manderic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, etc. These may be used in alone or in combination of two or more as described above.

In addition, the undecane or undecanal may make cosmetically or sitologically acceptable metal salts using a base. The alkali metal or alkaline earth metal salt may be obtained, for example, by dissolving the compound in an excess of alkali metal hydroxide or alkaline earth metal hydroxide solution, filtering the undissolved compound salt, and then evaporating and drying the filtrate. As the metal salts, it is particularly preferable to prepare sodium, potassium or calcium salts, but the metal salts are not limited thereto. Also, the corresponding silver salts may be obtained by reacting alkali metal or alkaline earth metal salts with suitable silver salts (e.g., silver nitrate).

The salts of undecane or undecanal may include all salts of acidic or basic groups that may be present in the compound of undecane or undecanal, unless otherwise indicated. Examples of the salts of undecane or undecanal may include sodium, calcium and potassium salts of a hydroxyl group, and examples of other cosmetically acceptable salts of an amino group may include hydrobromide, sulfate, hydrogen sulfate, phosphate, hydrogen phosphate, dihydrogen phosphate, acetate, succinate, citrate, tartrate, lactate, mandelate, methanesulfonate (mesylate) and p-toluenesulfonate (tosylate) salts etc.; and other cosmetically acceptable salts of an amino group may be prepared through known methods for preparing salts.

As used herein, "allergic disease" refers to diseases developed by an allergic reaction showing an excessive immune reaction in a body against an external antigen, and may be specifically one or more diseases selected from the group consisting of edema, anaphylaxis, allergic rhinitis, asthma, allergic conjunctivitis, allergic dermatitis, contact dermatitis, hives, pruritus, insect allergy, food allergy, and drug allergy, but is not limited thereto.

As used herein, "atopic dermatitis" is one of the allergic diseases, and is a skin disease accompanied by symptoms such as itchiness, dry skin, increased skin thickness, and characteristic eczema.

The pharmaceutical composition for preventing or treating allergic disease or atopic dermatitis according to the present disclosure is not particularly limited in the contents as long as it contains undecane, undecanal or pharmaceutically acceptable salts thereof, and preferably the dose of undecane or undecanal may be included in a concentration of 0.1 µM to 1000 µM, but is not limited thereto. At this time, if undecane or undecanal is less than the above concentration range, there is a problem in that it is difficult to exert a desirable preventive or therapeutic effect, and if undecane or undecanal exceed the above concentration range, there may be a risk of toxicity including cytotoxicity.

The pharmaceutical composition for preventing or treating allergic diseases or atopic dermatitis according to the present disclosure may be formulated and used in oral formulations such as powders, granules, tablets, capsules, suspensions, emulsions, syrups, or aerosols, external preparations, suppositories, or a form of a sterile injectable solution according to a conventional method, respectively, and may contain an appropriate carrier, excipient or diluent commonly used in the preparation of a pharmaceutical composition for formulation.

Examples of the carrier or excipient or diluent may include a variety of compounds or mixture thereof, including lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil. etc.

In the case of formulation, the formulation may be prepared by using diluents or excipients such as commonly used fillers, extenders, binders, wetting agents, disintegrants, surfactants, etc.

A solid formulation for oral administration may be prepared by mixing at least one excipient, for example, starch, calcium carbonate, sucrose or lactose, gelatin, etc., with the undecane or undecanal. Also, in addition to simple excipients, lubricants such as magnesium stearate and talc may be used.

Examples of a liquid formulation for oral administration include suspensions, oral liquids, emulsions, syrups, etc., and include various excipients, for example, wetting agents, sweeteners, aromatics, preservatives, etc., in addition to water and liquid paraffin which are simple diluents commonly used.

Examples of formulations for parenteral administration include sterilized aqueous solutions, non-aqueous solutions, suspensions, emulsions, lyophilized formulations, and suppositories. Examples of the non-aqueous solvent and suspending agent may include propylene glycol, polyethylene glycol, vegetable oil such as olive oil, and injectable ester such as ethyl oleate. Examples of a base for suppositories may include witepsol, macrogol, Tween 61, cacao butter, laurin, glycerol gelatin, etc.

A preferred dosage of the pharmaceutical composition for preventing or treating allergic disease or atopic dermatitis according to the present disclosure is varied with state and weight of a patient, severity of diseases, drug forms, an administration route and a duration, but may be properly selected by one skilled in the art. However, for a desirable effect, it may be administered at 0.0001 to 2,000 mg/kg per day, and preferably 0.001 to 2,000 mg/kg. The administration may be carried out once a day, or may be divided several times. However, the scope of the present disclosure is not limited by the dosage.

The pharmaceutical composition for preventing or treating allergic diseases or atopic dermatitis according to the present disclosure may be administered to mammals such as mouse, mice, livestock, and humans by various routes. All modes of administration may be, for example, oral, rectal or intravenous, intramuscular, subcutaneous, intrauterine dural, or intracerebroventricular inj ection.

The composition containing the active ingredient, according to the present disclosure may alleviate and prevent allergic diseases or atopic dermatitis symptoms by reducing the expression of IL-4, IL-13, TNF-α, IL-1β, IL-6, or IL-8 that are excessively secreted due to an immune response.

In one embodiment of the present disclosure, as a result of treatment with undecane or undecanal, it was confirmed that the expression of IL-4, IL-13, TNF-α, IL-1β, IL-6, and IL-8, which are inflammatory cytokines secreted by the immune response of cells was remarkably reduced, and that undecane or undecanal may suppress an excessive immune response and treat inflammation (FIGS. 1 to 4).

Also, the present disclosure provides a composition for preventing or alleviating allergic diseases or atopic dermatitis, containing, as an active ingredient, one or more selected from the group consisting of undecane, undecanal, and pharmaceutically acceptable salts thereof.

The specific content of the undecane and undecanal is the same as described above.

The composition for preventing or alleviating allergic diseases or atopic dermatitis of the present disclosure may be a health functional food composition, a cosmetic composition, or a fragrance composition.

As used herein, the term "health functional food" refers to food manufactured and processed in the form of tablets, capsules, powders, granules, liquids, pills, etc., using raw materials or ingredients having useful functions for the human body. Here, the term "functionality" refers to obtaining useful effects for health purposes such as regulating nutrients or physiological effects on the structure and function of the human body. The health functional food of the present disclosure may be manufactured by a method commonly used in the art, and at the time of manufacture, may be prepared by adding raw materials and components commonly added in the art. In addition, the formulation of the health functional food may be prepared without limitation as long as it is a formulation recognized as a health functional food. The health functional food composition according to the present disclosure has the advantage that there are no side effects that may occur when taking the drug for a long period of time by using food as a raw material, unlike general drugs, and has excellent portability, and may be taken as an adjuvant for enhancing the anti-allergic effect or the effect of alleviating atopic dermatitis symptoms.

In the health functional food for preventing or alleviating allergic diseases or atopic dermatitis according to the present disclosure, when undecane or undecanal is used as an additive in health functional food, the undecane or undecanal may be added by itself or may be used with other food or food ingredients, and may be appropriately used according to a conventional method. The mixing amount of the active ingredient may be appropriately determined according to each purpose of use, such as prevention, health or treatment.

The formulation of health functional food may be not only in the form of powders, granules, pills, tablets, and capsules, but also in the form of general food or beverages.

The type of food is not particularly limited, and examples of food to which the substance can be added include meat, sausage, bread, chocolate, candy, snacks, confectionery, pizza, ramen, other noodles, gums, dairy products including ice cream, various soups, beverages, teas, drinks, alcoholic beverages, and vitamin complexes, and may include all foods in a conventional sense.

In general, in the production of food or beverage, the undecane or undecanal may be added in an amount of 15 parts by weight or less, preferably 10 parts by weight or less, based on 100 parts by weight of the raw material. However, in the case of long-term intake for health and hygiene or health control, the amount may be not more than the above range, and since the present disclosure uses a fraction from a natural product and thus there is no problem in terms of safety, amounts not less than the above range may also be used.

Beverages among functional foods according to the present disclosure may contain various flavoring agents or natural carbohydrates as additional ingredients, like conventional beverages. The above-mentioned natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As the sweetener, natural sweeteners such as taumatine and stevia extract, synthetic sweeteners such as saccharin and aspartame, and the like may be used. The ratio of the natural carbohydrate may be about 0.01 to 0.04 g, and preferably about 0.02 to 0.03 g per 100 mL of the beverage according to the present disclosure.

In addition to those described above, the health functional food for preventing or alleviating allergic diseases or atopic dermatitis according to the present disclosure may include various nutrients, vitamins, electrolytes, flavoring agents, coloring agents, pectic acid and a salt thereof, alginic acid and a salt thereof, organic acids, and protective colloids thickening agents, pH adjusting agents, stabilizing agents, preservatives, glycerin, alcohols, carbonating agents used in carbonated beverages. In addition, the health functional food composition for preventing or alleviating allergic diseases or atopic dermatitis according to the present disclosure may contain flesh for the production of natural fruit juice, fruit juice beverage, and vegetable beverage. These components may be used independently or in combination. The ratio of these additives is not limited, but is generally selected in the range of 0.01 to 0.1 parts by weight relative to 100 parts by weight of the functional food of the present disclosure.

As used herein, the term "cosmetic composition" refers to a composition containing the compound, and the formulation may be in any form. Examples of cosmetics prepared using the composition of such a formulation include creams such as nourishing creams, eye creams, massage creams, and cleansing creams, packs, lotions such as nourishing lotions, essences, toners such as softening toner and nourishing toner, powders, foundations, and makeup bases, and may be manufactured and commercialized in any form of these formulations in order to achieve the object of the present disclosure, and is not limited to the above examples. In addition, the cosmetic composition according to the present disclosure may be formulated by a conventional cosmetic preparation method. Specifically, the cosmetic of the present disclosure may have any one formulation selected from the group consisting of skin lotion, skin softener, skin toner, astringent, lotion, milk lotion, moisture lotion, nourishing lotion, massage cream, nourishing cream, moisture cream, hand cream, essence, pack, mask pack, mask sheet, soap, shampoo, cleansing foam, cleansing lotion, cleansing cream, body lotion, body cleanser, emulsion, press powder, loose powder, and eye shadow.

The cosmetic composition according to the present disclosure may include other additives such as excipients, carriers, etc., in addition to undecane, undecanal or salts thereof, and be applied and blended as many common ingredients as necessary in general skin cosmetics.

Specifically, the cosmetic composition according to the present disclosure may further contain a transdermal penetration enhancer. As used herein, the term transdermal penetration enhancer is a composition that allows a desired component to penetrate into the blood vessel cells of the skin at a high absorption rate. Preferably, other phospholipid components, liposome components, etc., used in lecithin cosmetics are contained, but are not limited thereto.

In addition, examples of the oil that may be mainly used as an oily component may include one or more selected from vegetable oil, mineral oil, silicone oil, and synthetic oil. More specific examples thereof may include mineral oil, cyclomethicone, squalane, octyldodecyl myristate, olive oil, vitis vinifera seed oil, macadamia nut oil, glyceryl octanoate, castor oil, ethylhexyl isononanoate, dimethicone, cyclopentasiloxane, and sunflower seed oil.

In addition, 0.1 to 5% by weight of a surfactant, a higher alcohol, etc., may be added to enhance the emulsifying ability. Examples of such surfactants may include conventional surfactants such as nonionic surfactants, anionic surfactants, cationic surfactants, amphoteric surfactants, and phospholipids, etc., and specific examples there may include sorbitan sesquinoleate, polysorbate 60, glyceryl stearate, lipophilic glyceryl stearate, sorbitan oleate, sorbitan stearate, DEA-cetyl phosphate, sorbitan stearate/sucrose cocoate, glyceryl stearate/polyethylene glycol-100 stearate, ceteareth-6 olivate, arachidyl alcohol/behenyl alcohol/arachidyl glucoside, polypropylene glycol-26-buteth-26/polyethylene glycol-40 hydrogenated castor oil, etc. Examples of the higher alcohol may include an alcohol having 12 to 20 carbon atoms, such as cetyl alcohol, stearyl alcohol, octyldodecanol, isostearyl alcohol, etc., in alone or in combination of one or more as described above.

0.001% to 5% by weight of one or more thickeners such as carbomer, xanthan gum, bentonite, magnesium aluminum silicate, cellulose gum, dextrin palmitate, and the like in order to adjust the viscosity or hardness of the aqueous phase, may be further added to the aqueous phase component

In addition, medicinal ingredients such as higher fatty acids, vitamins etc., and sunscreens, antioxidants (butylhydroxyanisole, propyl gallic acid, elisorbic acid, tocopheryl acetate, butylated hydroxy toluene, etc.), preservatives (methylparaben, butylparaben, propylparaben, phenoxyethanol, imidazolidinylurea, chlorphenesin, etc.), colorants, pH adjusters (triethanolamine, citric acid, sodium citrate, malic acid, sodium malate, fumaric acid, sodium fumarate, succinic acid, sodium succinate, sodium hydroxide, sodium monohydrogen phosphate, etc.), humectants (glycerin, sorbitol, propylene glycol, butylene glycol, hexylene glycol, diglycerin, betaine, glycereth-26, methylgluceth-20, etc.), lubricants, etc., may be further added in the cosmetic composition according to the present disclosure, if necessary.

In addition, the cosmetic composition according to the present disclosure further contains a material that can supplementally provide essential nutrients to the skin, and preferably contains an auxiliary agent including, but not limited to, natural fragrance, cosmetic fragrance, or herbal medicine.

The effective content of undecane, undecanal, or cosmetically acceptable salts thereof in the cosmetic composition according to the present disclosure is not particularly limited, and may be contained in an amount of 0.0001% to 20% by weight, based on the total weight of the composition. Less than 0.0001% by weight of undecane, undecanal, or salts thereof in the cosmetic may have no wrinkle-improving effect because the dose is small, and undecane, undecanal, or salts thereof in an amount of more than 20% by weight may exhibit previously known toxicity.

As used herein, the term "fragrance composition" may be combined with skin external bases such as perfumes, cosmetics, bathing agents, food, pharmaceuticals, etc., and the combined amount may be appropriately selected for a combination to achieve the desired effect according to conventional techniques in the art.

The formulation of the fragrance composition according to the present disclosure is not particularly limited, and may be any one selected from powder, granule, liquid spray, solid and gel type formulations.

The fragrance composition can be used to manufacture cosmetic products containing perfume, soap cleaning products containing bath soap, indoor cleaning products containing glass cleaners, perfume products containing automobile air fresheners, bath products containing herbal type bath products, and perfume products including stationery, environmental products containing office air fresheners, or industrial products containing synthetic resins.

The fragrance composition according to the present disclosure contains undecane or undecanal as an active ingredient thereof in a range of 0.00001% to 10% by weight, preferably 0.00001% to 1.0% by weight, and more preferably from 0.00001% to 0.5% by weight, depending on the product type in which the perfume composition according to the present disclosure is embodied, based on the total weight of the fragrance composition according to the present disclosure.

The product form in which the fragrance composition is embodied, includes, but is not limited to, soap, cosmetics, bathing agent, aroma oil, etc., specifically, body lotion, shampoo, hair rinse, hair conditioner, hair treatment, antiperspirant, skin lotion, skin cream, deodorant, perfumes (sprays or fumigants), lipsticks, lip creams, bath products, etc.

These products may include various additives such as blood circulation promoters, antiinflammatory agents, moisturizing agents, astringents, inorganic salts, organic salts, oily ingredients, surfactants, herbal medicines, colorants, fragrances, sulfur, sinter deposits, bactericides, etc.

In particular, the fragrance composition according to the present disclosure will be generally used as an external preparation for the skin of cosmetic formulations such as make-up products, skin lotions, and skin creams, and in this case, may contain ingredients commonly used in these cosmetic formulations.

In particular, the fragrance composition according to the present disclosure is preferably used by being incorporated into a bathing agent in that undecane or undecanal as the active ingredient has an activity of alleviating atopic dermatitis. In this case, however, the active ingredient may be included in an amount of preferably 0.00001 to 1% by weight, and more preferably 0.0001 to 0.1% by weight, based on the total weight of the bath agent. When the fragrance composition according to the present disclosure is incorporated into a bath agent, the bath agent may be added to the bath water at a concentration of 0.015 to 15 ppm.

The bath agent may contain an inorganic salt, an organic acid, an oily component, etc., in addition to the active ingredient of the fragrance composition according to the present disclosure.

Inorganic salts may be exemplified by sodium chloride, sodium hydrogen carbonate, sodium carbonate, borax, sodium sulfate, sodium sulfide, sodium sesquicarbonate, sodium nitrate, sodium thiosulfate, sodium polyphosphate, sodium phosphate, calcium oxide, magnesium oxide, calcium carbonate, magnesium carbonate, potassium chloride, sulfide potassium, etc., and these may be used in alone or in combination of two or more as described above. These inorganic salts may be added to the bath agent in an amount of 5 wt% or more, preferably 10 wt% or more, based on the total weight of the bath agent.

The organic acid may be exemplified by succinic acid, fumaric acid, malic acid, tartaric acid, citric acid, benzoic acid, and the like, and these may be used in alone or in combination of two or more as described above. These organic acids may be added to the bath agent in the range of 0.1 to 50% by weight, based on the total weight of the bath agent.

Examples of the oily component include waxes, hydrocarbons, higher fatty acids, higher alcohols, esters, silicone oils, etc.

The bath agent may further contain other ingredients commonly used in the art. Examples of such components include inorganic acids such as boric acid, metasilicic acid, and silicic anhydride; herbal powders such as fennel, ginkgo biloba, ginger, citrus peel, valerian root, mint, ginseng, and oats; natural pigments found to be harmless to the human body, such as coal tar dye, chlorophyll, riboflavin, saffron, and anthraquinone; vitamins such as vitamin A, vitamin C, vitamin D, and vitamin E; sulfur, mica powder, white clay powder, loess powder, rice bran carbide, disinfectant, preservative, etc.

Such a bath agent may be prepared in any form such as granules, tablets, liquids, powders, etc.

Also, the present disclosure provides a pharmaceutical composition, a quasi-drug, and a cosmetic composition for curing skin wound or promoting skin regeneration, containing, as an active ingredient, one or more selected from the group consisting of undecane, undecanal, and pharmaceutically acceptable salts thereof.

Hereinafter, the present disclosure will be described in more detail through examples. These examples are merely for illustrative purposes the present disclosure, and it will be apparent to those of ordinary skill in the art that the scope of the present disclosure is not construed as being limited by these examples.

### Example 1: Evaluation of secretion and expression of inflammation-related cytokines after treatment with undecane on mast cells and keratinocytes

### 1-1. Experimental method

### 1) Mast cell culture

Mast cells (rat basophilic leukemia cell line, RBL-2H3) were purchased from ATCC (Manassas, VA, USA) and used. Cells were cultured in the culture medium of DMEM (Dulbecco modified eagle medium) (Gibco BRL, USA) containing 10% heat-inactivated feta bovine serum (FBS) (Gibco BRL, USA) and 1% penicillin and streptomycin (Gibco BRL, USA). Cells were tested by culturing at 37°C, 5% CO₂ conditions. Mast cells were suspended in DMEM containing 10% FBS and then dispensed to be a cell number of 1×10⁶ cells/ml in a 6-well plate (Corning, USA).

Then, it was sensitized with anti-DNP (dinitrophenyl)-IgE (30 ng/ml) and cultured for 24 hours at 37°C, 5% CO₂ in an incubator. After washing twice with PBS (phosphate buffered saline), DNP-HSA (dinitrophenylated human serum albumin; 10 µg/ml) was treated for 4 hours to induce an immune response. In addition, in order to evaluate atopy alleviating effect by undecane, mast cells treated with anti-DNP (dinitrophenyl)-IgE and cultured for 24 hours were treated with 100 µM of undecane, cultured for 1 hour, and then an immune response was induced with DNP-HSA. Normal cells were treated with DMSO instead of undecane under the same conditions as undecane-treated cells, and positive control cells were treated with 10 µM of tacrolimus instead of undecane and cultured for 1 hour, and then, an immune response was induced with DNP-HSA.

### 2) Keratinocyte cell culture

Keratinocytes (human keratinocyte cell line, HaCaT) were purchased from ATCC (Manassas, VA, USA) and used. For cell culture, the culture medium of DMEM containing 10% fetal bovine serum (FBS) and antibiotics was used. The culture vessel used a 75T-flask and a 6-well plate, and cultured in a 37°C incubator supplied with 5% CO₂. The culture medium was changed every 3 to 4 days, and subculture was performed when the cells were excessively proliferated. The dispensed HaCaT cells (5×10⁵/well) were cultured for 24 hours and then washed with PBS. In order to induce an immune response to keratinocytes, TNF-α (tumor necrosis factor-α) (10 ng/ml) and IFN-γ (interferon gamma) (10 ng/ml) were treated together in DMEM medium without FBS and incubated for 24 hours. At this time, in order to evaluate the efficacy of alleviating the immune response of undecane, 100 µM of undecane was simultaneously treated, and in the case of normal cells, DMSO instead of undecane, and in the case of positive control cells, 10 µM of tacrolimus instead of undecane were treated simultaneously with the immune response inducer and incubated for 24 hours.

### 3) RNA isolation and real-time PCR (quantitative reverse-transcription polymerase chain reaction) using Trizol method

After removing the supernatant of the mast cells that induced the immune response, 1 ml of trizol was added, left for 2 minutes, and chloroform was added, vortexed for 10 seconds, centrifuged at 5,000 rpm for 10 minutes, and the upper layer was taken, and mixed with the same amount of isopropanol and shaken. The supernatant was removed by centrifugation at 13,000 rpm for 25 minutes, and the pellet was dissolved in 20 µl of diethyl pyrocarbonate (DEPC)-DW and stored at -20°C and then used in the experiment. RT-PCR was performed using a one-step RT-PCR PreMixkit (iNtRON Biotechnology, Korea) at 45°C for 30 minutes and at 94°C for 5 minutes, followed by denaturation at 94°C for 30 seconds, and then after annealing at 55°C for 30 seconds, the cycle of extension at 72°C for 1 minute was repeated 32 times, and the last extension was performed at 72°C for 5 minutes, and PiQ SYBR green supermix (Bio-Rad) and CFX Connect^{™} Real-Time PCR Detection System (Bio-Rad) was used to perform quantitative PCR, and the primer sequences used are as shown in Table 1.

**[Table 1]**

| Primer sequence used in RT-PCR | | | | |
|---|---|---|---|---|
| Gene description | Primers | Sequences (5'→3') | Annealing temperature (°C) | PCR product (bp) |
| IL-4 | F | CAGGGTGCTTCGCAAATT TTAC | 55 | 104 |
| | R | ACCGAGAACCCCAGACTT GTT | | |
| IL-13 | F | GGTGGCCTCACCTCCCCA AG | 60 | 234 |
| | R | GATGACACTGCAGTTGGA GATGCTG | | |
| TNF-α | F | CAGCCGATTTGCCACTTC ATA | 60 | 168 |
| | R | TCCTTAGGGCAAGGGCTC TT | | |
| IL-1β | F | TTACAGTGGCAATGAGGA TGA | 57 | 250 |
| | R | TGTAGTGGTGGTCGGAGA TT | | |
| IL-6 | F | CCTGAGAAAGGAGACAT GTAACAAGA | 58 | 460 |
| | R | TGGAAGGTTCAGGTTGTT TTCTG | | |
| IL-8 | F | CTGGCCGTGGCTCTCTTG | 65 | 292 |
| | R | TTAGCACTCCTTGGCAAA ACTG | | |
| β-actin | F | ACCGTGAAAAGATGACCC AG | 60 | 619 |
| | R | TGTCAGCTGTGGTGGTGA AG | | |

### 1-2. Experimental result

### 1) Changes of inflammatory cytokine production in mast cells

In control cells wherein they were sensitized with anti-DNP (dinitrophenyl)-IgE and wherein an immune response was induced by DNP-HSA, the expression of inflammatory cytokines IL-4, IL-13, and TNF-α was significantly increased compared to that of normal mast cells. On the other hand, as a result of treatment with undecane or tacrolimus while inducing an immune response by anti-DNP (dinitrophenyl)-IgE and DNP-HSA, the expression of the inflammatory cytokines (IL-4, IL-13, and TNF-α) increased due to the immune response were all significantly reduced (FIG. 1).

### 2) Changes of inflammatory cytokine production in keratinocytes

The expression of inflammatory cytokines IL-1β, IL-6, and IL-8 was significantly increased in control cells wherein an immune response was induced by TNF-α and IFN-γ, compared to normal keratinocytes. On the other hand, as a result of treating keratinocytes with undecane or tacrolimus together with TNF-α and IFN-γ,the expression of all inflammatory cytokines (IL-1β, IL-6, and IL-8) increased due to an immune response was significantly reduced (FIG. 2).

### Example 2: Evaluation of secretion and expression of inflammation-related cytokines after treatment with undecanal on mast cells and keratinocytes

### 2-1. Experimental method

### 1) Mast cell culture

Mast cells (rat basophilic leukemia cell line, RBL-2H3) were purchased from ATCC (Manassas, VA, USA) and used. Cells were cultured in the culture medium of DMEM (Dulbecco modified eagle medium) (Gibco BRL, USA) containing 10% heat-inactivated feta bovine serum (FBS) (Gibco BRL, USA) and 1% penicillin and streptomycin (Gibco BRL, USA). Cells were tested by culturing at 37°C, 5% CO₂ conditions. Mast cells were suspended in DMEM containing 10% FBS and then dispensed to be a cell number of 1×106 cells/ml in a 6-well plate (Corning, USA).

Then, it was sensitized with anti-DNP (dinitrophenyl)-IgE (30 ng/ml) and cultured for 24 hours at 37°C, 5% CO₂ in an incubator. After washing twice with PBS (phosphate buffered saline), DNP-HSA (dinitrophenylated human serum albumin; 10 µg/ml) was treated for 4 hours to induce an immune response. In addition, in order to evaluate an effect of alleviating atopy by undecanal, mast cells treated with anti-DNP (dinitrophenyl)-IgE and cultured for 24 hours were treated with 100 µM of undecanal, cultured for 1 hour, and then an immune response was induced with DNP-HSA. Normal cells were treated with DMSO instead of undecanal under the same conditions as undecanal-treated cells, and positive control cells were treated with 10 µM of tacrolimus instead of undecanal and cultured for 1 hour, and then, an immune response was induced with DNP-HSA.

### 2) Keratinocyte cell culture

Keratinocytes (human keratinocyte cell line, HaCaT) were purchased from ATCC (Manassas, VA, USA) and used. For cell culture, the culture medium of DMEM containing 10% fetal bovine serum (FBS) and antibiotics was used. The culture vessel used a 75T-flask and a 6-well plate, and cultured in a 37°C incubator supplied with 5% CO₂. The culture medium was changed every 3 to 4 days, and subculture was performed when the cells were excessively proliferated. The dispensed HaCaT cells (5×10⁵/well) were cultured for 24 hours and then washed with PBS. In order to induce an immune response to keratinocytes, TNF-α (tumor necrosis factor-α) (10 ng/ml) and IFN-γ (interferon gamma) (10 ng/ml) were treated together in DMEM medium without FBS and incubated for 24 hours. At this time, in order to evaluate the efficacy of alleviating the immune response of undecanal, 100 µM of undecanal was simultaneously treated, and in the case of normal cells, DMSO instead of undecanal, and in the case of positive control cells, 10 µM of tacrolimus instead of undecanal were treated simultaneously with the immune response inducer and incubated for 24 hours.

### 3) RNA isolation and real-time PCR (quantitative reverse-transcription polymerase chain reaction) using Trizol method

RNA isolation and real-time PCR (quantitative reverse-transcription polymerase chain reaction) using a Trizol method was performed by the same method as 3) RNA isolation using the Trizol method and real-time PCR of 1-1. of Example 1 described above.

### 2-2. Experimental result

### 1) Changes of inflammatory cytokine production in mast cells

In control cells wherein they were sensitized with anti-DNP (dinitrophenyl)-IgE and wherein an immune response was induced by DNP-HSA, the expression of inflammatory cytokines IL-4, IL-13, and TNF-α was significantly increased compared to that of normal mast cells. On the other hand, as a result of treatment with undecanal or tacrolimus while inducing an immune response by anti-DNP (dinitrophenyl)-IgE and DNP-HSA, the expression of the inflammatory cytokines (IL-4, IL-13, and TNF-α) increased due to the immune response were all significantly reduced (FIG. 3).

### 2) Changes of inflammatory cytokine production in keratinocytes

The expression of inflammatory cytokines IL-1β, IL-6, and IL-8 was significantly increased in control cells wherein an immune response was induced by TNF-α and IFN-γ, compared to normal keratinocytes. On the other hand, as a result of treating keratinocytes with undecanal or tacrolimus together with TNF-α and IFN-γ,the expression of all inflammatory cytokines (IL-1β, IL-6, and IL-8) increased due to an immune response was significantly reduced (FIG. 4).

### [Mode for Disclosure]

Hereinafter, an example of the preparation of a pharmaceutical, food or cosmetic containing undecane or undecanal as an active ingredient according to the present disclosure will be described, but the present disclosure is not intended to be limited thereto, but only to be described in detail. The pharmaceutical, food or cosmetic composition of Preparation Examples 1 to 3 according to the following composition ingredients and composition ratios, with ingredients excellent in the prevention and treatment (or prevention and alleviation effect) of the allergic disease or atopic dermatitis was prepared according to a conventional method.

### [Preparation Example 1] Preparation of pharmaceutical composition

### <1-1> Preparation of powder

20 mg of undecane or undecanal
100 mg of lactose hydrate
10 mg of talc

The above ingredients were mixed and filled in an airtight cloth to prepare a powder.

### <1-2> Preparation of tablets

10 mg of undecane or undecanal
100 mg of corn starch
100 mg of lactose hydrate
2 mg of magnesium stearate

After mixing the above ingredients, tablets were prepared by tableting according to a conventional method of preparing tablets.

### <1-3> Preparation of capsules.

10 mg of undecane or undecanal
3 mg of microcrystalline cellulose
14.8 mg of lactose hydrate
0.2 mg of magnesium stearate

After mixing the above ingredients, the capsules were prepared by filling in gelatin capsules according to a conventional method of preparing capsule.

### <1-4> Preparation of injection

10 mg of undecane or undecanal
180 mg of mannitol
2974 mg of sterile distilled water for injection
26 mg of sodium monohydrogen phosphate

After mixing the above ingredients, the content of the above components per 1 ampoule (2 mL) was prepared according to a conventional method for preparing injections.

### <1-5> Preparation of liquid preparation

10 mg of undecane or undecanal
10 mg of isomerized sugar
5 mg of mannitol
an appropriate amount of purified water
an appropriate amount of lemon flavor

The above components are dissolved by adding each component to purified water according to a conventional preparation method, and after adding an appropriate amount of lemon flavor, purified water is added to adjust the total volume to 100 mL, sterilized, and filled in a brown bottle to prepare a solution.

### [Preparation Example 2] Preparation of health food

### <2-1> Preparation of health supplements

10 mg of undecane or undecanal
an appropriate amount of vitamin mixture
70 µg of vitamin A acetate
1.0 mg of vitamin E
0.13 mg of vitamin B₁
0.15 mf of vitamin B₂
0.5 mg of vitamin B₆
0.2 µg of vitamin B₁₂
10 mg of vitamin C
10 µg of biotin
1.7 mg of nicotinic acid amide
50 µg of folic acid
0.5 mg of calcium pantothenate
an appropriate amount of mineral mixture
1.75 mg of ferrous sulfate
0.82 mg of zinc oxide
25.3 mg of magnesium carbonate
15 mg of potassium monophosphate
55 mg of dibasic calcium phosphate
30 mg of potassium citrate
100 mg of calcium carbonate
24.8 mg of magnesium chloride

The composition ratio of the vitamin and mineral mixture was made by mixing ingredients relatively suitable for health food in a preferred embodiment, but the mixing ratio may be arbitrarily modified, and the ingredients are mixed according to a conventional method of preparing health food, and then the granule may be prepared, and may be used to prepare a health food composition according to a conventional method.

### <2-2> Preparation of health drinks

10 mg of undecane or undecanal
15 g of vitamin C
100 g of vitamin E (powder)
19.75 g of iron lactate
3.5 g of zinc oxide
3.5 g of nicotinic acid amide
0.2 g of vitamin A
0.25 g of vitamin B₁
0.3 g of vitamin B₂
quantification of purified water

After mixing the above ingredients according to a conventional method of preparing health drink, and then stirring and heating at 85°C for about 1 hour, the resulting solution was filtered and obtained in a sterilized 2L container, sealed and sterilized, then refrigerated and stored and used to prepare the health drink compositions according to the present disclosure.

The composition ratio was made by mixing ingredients relatively suitable for favorite drink in a preferred embodiment, but the mixing ratio may be arbitrarily modified according to regional and national preferences such as demand class, demanding country, use, etc.

### [Preparation Example 3] Preparation of a cosmetic composition

Hereinafter, preparation example of a cosmetic composition containing the extract of the present disclosure will be described, but the present disclosure is not intended to be limited thereto, but only to be described in detail.

### <3-1> Nutrient lotion (milk lotion)

2.0% by weight of undecane or undecanal
5.0% by weight of squalane
4.0% by weight of beeswax
1.5% by weight of Polysorbate 60
1.5% by weight of sorbitan sesquioleate
0.5% by weight of liquid paraffin
5.0% by weight of caprylic/capric triglycerides
3.0% by weight of glycerin
3.0% by weight of butylene glycol
3.0% by weight of propylene glycol
0.1% by weight of carboxy vinyl polymer
0.2% by weight of triethanolamine
an appropriate amount of preservatives, colors, and fragrances
Purified water to 100% by weight

The mixing ratio was made by mixing ingredients relatively suitable for nourishing toner in a preferred embodiment, but may be arbitrarily modified, and the nutrient lotion (milk lotion) may be prepared according to a conventional prepararation method in the cosmetic field.

### <3-2> Softening lotion (skin lotion)

2.0% by weight of undecane or undecanal
3.0% by weight of glycerin
2.0% by weight of butylene glycol
2.0% by weight of propylene glycol
0.1% by weight of carboxy vinyl polymer
0.2% by weight of PEG 12 nonyl phenyl ether
0.4% by weight of Polysorbate 80
10.0% by weight of ethanol
0.1% by weight of triethanol amine
an appropriate amount of preservatives, colors, and fragrances
purified water to 100% by weight

The mixing ratio was made by mixing ingredients relatively suitable for softening toner in a preferred embodiment, but may be arbitrarily modified, and the softening lotion (skin lotion) may be prepared according to a conventional preparation method in the cosmetic field.

### <3-3> Nourishing Cream

2.0% by weight of undecane or undecanal
1.5% by weight of Polysorbate 60
1.5% by weight of sorbitan sesquioleate
2.0% by weight of PEG60 harden castor oil
10% by weight of liquid paraffin
5.0% by weight of squalane
5.0% by weight of caprylic/capric triglycerides
5.0% by weight of glycerin
3.0% by weight of butylene glycol
3.0% by weight of propylene glycol
0.2% by weight of triethanol amine
an appropriate amount of preservative
an appropriate amount of color
an appropriate amount of fragrance
purified water to 100% by weight

The mixing ratio was made by mixing ingredients relatively suitable for nourishing cream in a preferred embodiment, but the mixing ratio may be arbitrarily modified, and the nourishing cream may be prepared according to a conventional preparation method in the cosmetic field.

### <3-4> Massage cream

1.0% by weight of undecane or undecanal
10.0% by weight of beeswax
1.5% by weight of Polysorbate 60
0.2% by weight of PEG 60 harden castor oil
0.8% by weight of sorbitan sesquioleate
40.0% by weight of liquid paraffin
5.0% by weight of squalane
4.0% by weight of caprylic/capric triglycerides
5.0% by weight of glycerin
3.0% by weight of butylene glycol
3.0% by weight of propylene glycol
0.2% by weight of triethanol amine
an appropriate amount of preservatives, colors, and fragrances
purified water to 100% by weight

The mixing ratio was made by mixing ingredients relatively suitable for massage cream in a preferred embodiment, but the mixing ratio may be arbitrarily modified, and the massage cream may be prepared according to a conventional method in the cosmetic field.

### <3-5> Pack

1.0% by weight of undecane or undecanal
13.0% by weight of polyvinyl alcohol
0.2% by weight of sodium carboxymethyl cellulose
5.0% by weight of glycerin
0.1% by weight of allantoin
6.0% by weight of ethanol
0.3% by weight of PEG 12 nonyl phenyl ether
0.3% by weight of Polysorbate 60
an appropriate amount of preservatives, colors, and fragrances
purified water to 100% by weight

The mixing ratio was made by mixing ingredient relatively suitable for packs in a preferred embodiment, but the mixing ratio may be arbitrarily modified, and the pack may be prepared according to a conventional preparation method in the cosmetic field.

### <3-6> Gel

0.5% by weight of undecane or undecanal
0.05% by weight of sodium ethylenediamine acetate
5.0% by weight of glycerin
0.3% by weight of carboxy vinyl polymer
5.0% by weight of ethanol
0.5% by weight of PEG 60 harden castor oil
0.3% by weight of triethanol amine
an appropriate amount of preservatives, colors, and fragrances
purified water to 100% by weight

The mixing ratio was made by mixing ingredients relatively suitable for gel in a preferred embodiment, but may be arbitrarily modified, and the gel may be prepared according to a conventional preparation method in the cosmetic field.

The mixing ratio was made by mixing ingredient relatively suitable for a cosmetic composition in a preferred embodiment, but may be applied to cosmetics for various uses, including other color cosmetics, and depending on its efficacy, may be used in the preparation of a drug that may be applied thinly to the human body, that is, an ointment. Also, the mixing ratio may be arbitrarily modified according to regional and national preferences such as the demanding class, demanding country, use, etc.

The description of the present disclosure above is for illustrative purposes only, and one skilled in the art to which the present disclosure pertains will understand that it is possible to easily transform it into other specific forms without changing the technical spirit or essential features of the present disclosure. Therefore, it should be understood that the embodiments described above are illustrative in all respects and not limiting.

## Claims

1. A pharmaceutical composition for preventing or treating allergic diseases or atopic dermatitis, comprising, as an active ingredient, one or more selected from the group consisting of undecane, undecanal, and pharmaceutically acceptable salts thereof.

2. The pharmaceutical composition of claim 1, wherein the allergic diseases include one or more selected from the group consisting of edema, anaphylaxis, allergic rhinitis, asthma, allergic conjunctivitis, allergic dermatitis, contact dermatitis, urticaria, pruritus, insect allergy, food allergy, and drug allergy.

3. The pharmaceutical composition of claim 1, wherein the active ingredient reduces the expression of IL-4, IL-13, TNF-α, IL-1β, IL-6, or IL-8.

4. A composition for preventing or alleviating allergic diseases or atopic dermatitis, comprising, as an active ingredient, one or more selected from the group consisting of undecane, undecanal, and pharmaceutically acceptable salts thereof.

5. The composition of claim 4, wherein the composition is a functional food composition, a cosmetic composition, or a fragrance composition.

6. A pharmaceutical composition for curing skin wound or promoting skin regeneration, comprising, as an active ingredient, one or more selected from the group consisting of undecane, undecanal and pharmaceutically acceptable salts thereof.

7. A quasi-drug composition for curing skin wound or promoting skin regeneration, comprising, as an active ingredient, one or more selected from the group consisting of undecane, undecanal and pharmaceutically acceptable salts thereof.

8. A cosmetic composition for curing skin wound or promoting skin regeneration, comprising, as an active ingredient, one or more selected from the group consisting of undecane, undecanal and pharmaceutically acceptable salts thereof.

9. A method for preventing or treating allergic diseases or atopic dermatitis comprising administering or taking a composition comprising, as an active ingredient, one or more selected from the group consisting of undecane, undecanal, and pharmaceutically acceptable salts thereof to a subject.

10. Use for preventing or treating allergic diseases or atopic dermatitis of a composition, comprising, as an active ingredient, one or more selected from the group consisting of undecane, undecanal, and pharmaceutically acceptable salts thereof.


<110>     BOTANICSENS

<120>     COMPOSITION INCLUDING UNDECANE OR UNDECANAL AS ACTIVE INGREDIENTS
          FOR ANTI-ALLERGY, IMPROVEMENT OF ATOPIC DERMATITIS, OR SKIN
          REGENERATION

<130>     PCT5040320

<150>     KR 10-2019-0003549
<151>     2019-01-10

<160>     14

<170>     KoPatentIn 3.0

<210>     1
<211>     22
<212>     DNA
<213>     Artificial Sequence

<220>
<223>     IL-4_F primer


<400>     1
cagggtgctt cgcaaatttt ac                                                22


<210>     2
<211>     21
<212>     DNA
<213>     Artificial Sequence

<220>
<223>     IL-4_R primer


<400>     2
accgagaacc ccagacttgt t                                                 21


<210>     3
<211>     20
<212>     DNA
<213>     Artificial Sequence

<220>
<223>     IL-13_F primer


<400>     3
ggtggcctca cctccccaag                                                   20


<210>     4
<211>     25
<212>     DNA
<213>     Artificial Sequence

<220>
<223>     IL-13_R primer

<400>    4
gatgacactg cagttggaga tgctg                                          25


<210>    5
<211>    21
<212>    DNA
<213>    Artificial Sequence

<220>
<223>    TNF-alpha_F primer


<400>    5
cagccgattt gccacttcat a                                              21


<210>    6
<211>    20
<212>    DNA
<213>    Artificial Sequence

<220>
<223>    TNF-alpha_R primer


<400>    6
tccttagggc aagggctctt                                                20


<210>    7
<211>    21
<212>    DNA
<213>    Artificial Sequence

<220>
<223>    IL-1beta_F primer


<400>    7
ttacagtggc aatgaggatg a                                              21


<210>    8
<211>    20
<212>    DNA
<213>    Artificial Sequence

<220>
<223>    IL-1beta_R primer


<400>    8
tgtagtggtg gtcggagatt                                                20


<210>    9
<211>    26
<212>    DNA
<213>    Artificial Sequence

<220>
<223>    IL-6_F primer

<400>    9
cctgagaaag gagacatgta acaaga                                          26


<210>    10
<211>    23
<212>    DNA
<213>    Artificial Sequence

<220>
<223>    IL-6_R primer


<400>    10
tggaaggttc aggttgtttt ctg                                             23


<210>    11
<211>    18
<212>    DNA
<213>    Artificial Sequence

<220>
<223>    IL-8_F primer


<400>    11
ctggccgtgg ctctcttg                                                   18


<210>    12
<211>    22
<212>    DNA
<213>    Artificial Sequence

<220>
<223>    IL-8_R primer


<400>    12
ttagcactcc ttggcaaaac tg                                              22


<210>    13
<211>    20
<212>    DNA
<213>    Artificial Sequence

<220>
<223>    beta-actin_F primer


<400>    13
accgtgaaaa gatgacccag                                                 20


<210>    14
<211>    20
<212>    DNA
<213>    Artificial Sequence

<220>
<223>    beta-actin_R primer

<400>    14
tgtcagctgt ggtggtgaag                                                    20